# EUROPÄISCHE PATENTSCHRIFT

(11) **EP 0 787 739 B1**
(45) Veröffentlichungstag und Bekanntmachung des Hinweises auf die Patenterteilung: **29.01.2003**
(21) Anmeldenummer: 96115414.3
(22) Anmeldetag: 26.09.1996
(51) Int. Cl.: C07H 15/207, C07D 207/16, C07D 211/62, A61K 31/70, A61K 31/40, A61K 31/435

(54) **Antiadhäsive Piperidin- und Pyrrolidin-Carbonsäuren**
Anti-adhesive piperidine and pyrollidine -carboxylic acids
Anto-adhésive piperidine et pyrollidine-acides carboxyliques

(30) Priorität: 09.10.1995 DE 19537334
(43) Veröffentlichungstag der Anmeldung: 06.08.1997
(73) Patentinhaber: Glycorex AB, 223 70 Lund (SE)
(72) Erfinder: Toepfer, Alexander, Dr., 65830 Kriftel (DE); Kretzschmar, Gerhard, Dr., 65760 Eschborn (DE); Schölkens, Bernward, Prof. Dr., 65779 Kelkheim (DE); Klemm, Peter, Dr., 52072 Aachen (DE); Hüls, Christoph, Dr., 55263 Wackernheim (DE); Seiffge, Dirk, Dr., 55246 Mainz-Kostheim (DE)
(74) Vertreter: Bjerre, Nils B.J.

(56) Entgegenhaltungen:
- EP-A- 0 671 409
- JOURNAL OF THE AMERICAN CHEMICAL SOCIETY, Bd. 117, Nr. 19, 17.Mai 1995, Seiten 5395-5936, XP000570082 TAKETO UCHIYAMA ET AL: "DESIGN AND SYNTHESIS OF SIALYL LEWIS X MIMETICS"
- JOURNAL OF BIOLOGICAL CHEMISTRY, Bd. 269, Nr. 31, 5.August 1994, Seiten 19663-19666, XP000569697 NARASINGA RAO B N ET AL: "SIALYL LEWIS ASPIRE MIMICS DERIVED FROM A PHARMACOPHORE SEARCH SELECTIN INHIBITORS WITH ANTI-INFLAMMATORY ACTIVITY"
- SYNLETT, Nr. 10, 1.Oktober 1994, Seiten 868-870, XP000569656 HANESSIAN S ET AL: "DESIGN AND SYNTHESIS OF GLYCOMIMETIC PROTOTYPES A MODEL SIALYL LEWISX LIGAND FOR E-SELECTIN"

## Beschreibung

Die Erfindung betrifft Konjugate, die aus ein- oder mehrfach carboxylierten Piperidin- bzw. Pyrrolidinderivaten und über eine Kette oder einen Cyclus verknüpfte Pyranosen, Furanosen oder Polyalkoholen bestehen. Die Carboxylgruppen der Piperidinderivate können entweder direkt am Ring sitzen oder über eine kurze Kette mit dem Ring verknüpft sein. Die Erfindung betrifft außerdem die Herstellung dieser Verbindungen sowie deren Verwendung zur Herstellung von Arzneimittel und Diagnostika.

Die Zirkulation von Blutzellen wie z.B. Leukozyten, Neutrophilen, Granulozyten und Monozyten ist auf molekularer Ebene ein vielstufiger, sehr komplexer Prozess, der nur in Teilschritten bekannt ist (Review: T.A.Springer, Cell 76, 301-314, 1994).

Jüngste Forschungsergebnisse zeigten, daß die in der Immunüberwachung entscheidende Rezirkulation der Lymphozyten sowie die Lokalisierung von Neutrophilen und Monozyten an Entzündungsherden sehr ähnlichen molekularen Mechanismen gehorchen. So kommt es bei akuten und chronischen Entzündungsprozessen zur Adhäsion der Leukozyten an Endothelzellen und Auswanderung in den Entzündungsherd und in die sekundären lymphatischen Organe.

An diesem Vorgang sind zahlreiche spezifische Signalmoleküle wie z.B. Interleukine, Leukotriene und Tumornekrosefaktor (TNF), deren G-Protein gekoppelte Rezeptoren und insbesondere gewebespezifische Zelladhäsionsmoleküle beteiligt, die eine genau kontrollierte Erkennung der Immun- und Endothelzellen gewährleisten. Zu den wichtigsten hierbei beteiligten Adhäsionsmolekülen, die im folgenden als Rezeptoren bezeichnet werden sollen, gehören die Selektine (E-, P- und L-Selektine), Integrine und die Mitglieder der Immunglobulin-Superfamilie.

Die drei Selektinrezeptoren bestimmen die Anfangsphase der Leukozytenadhäsion. E-Selektin wird auf Endothelzellen einige Stunden nach der Stimulierung beispielsweise durch Interleukin-1 (IL-1β) oder Tumornekrosefaktor (TNF-α) exprimiert, während P-Selektin in Blutplättchen und Endothelzellen gespeichert wird und nach Stimulierung durch Thrombin, Peroxidradikale oder Substanz P u.a. auf den Zelloberflächen präsentiert wird. L-Selektin wird dauerhaft auf Leukozyten exprimiert, wird im Verlauf der Entzündung jedoch rasch wieder von den Leukozyten abgespalten.

Die in der Anfangsphase entzündlicher Prozesse durch Selektin-Rezeptoren vermittelte Adhäsion von Leukozyten an Endothelzellen ist eine natürliche und notwendige Immunantwort auf diverse Entzündungsreize und Verletzungen des vasculären Gewebes.
Der Verlauf einer Reihe von akuten und chronischen Erkrankungen wird jedoch durch die übermäßige Adhäsion von Leukozyten und deren Infiltration in das betroffene Gerwebe sowie durch die Schädigung gesunden Gewebes im Sinne einer Autoimmunreaktion ungünstig beeinflußt. Dazu zählen beispielsweise Rheuma, Reperfusionsverletzungen wie myokardiale Ischämie/Infarkt (MI), akute Lungenentzündung nach operativem Eingriff, traumatischer Schock und Schlaganfall, Psoriasis, Dermatitis, ARDS (Atemnotsyndrom bei Erwachsenen) sowie die nach chirurgischen Eingriffen (Beispiel Angioplastie und By-Pass Operationen) auftretende Restenose.

Der natürliche Ligand mit der Struktur von SLeX wurde schon erfolgreich in Tierversuchen bei P-Selektin abhängigen Lungenverletzungen (M.S.Mulligan et al., Nature 1993, 364, 149) und bei myokardialen Reperfusionsverletzungen (M.Buerke et al., J.Clin.Invest. 1994, 93, 1140) verwendet. In ersten klinischen Prüfungen bei akuter Lungenentzündung soll die Verbindung in einer Dosis von 1-2 Gramm pro Tag und Patient eingesetzt werden (Mitteilung der Firma Cytel Corp,/ La Jolla (CA.) beim 2. Glycotechnology Meeting/CHI in La Jolla/USA am 16.-18. Mai 1994).

Diese hohe Wirkstoffdosis steht im Einklang mit der bekanntermaßen schwachen Affinität der natürlichen SLeX/A-Liganden zu den Selektin-Rezeptoren. So inhibiert SLeX in allen bekannten in vitro-Testsystemen die Zelladhäsion an Selektinrezeptoren erst bei einer relativ hohen Konzentration im Bereich von IC₅₀ ca.1 mM (Jacob et al., Biochemistry 1995, 34, 1210). In einigen Publikationen und Patentanmeldungen wurde inzwischen über Bemühungen berichtet, durch strukturelle Variation des Liganden zu fester bindenden Antagonisten zu gelangen. Ziel dieser Arbeiten ist die Bereitstellung wirksamerer Antagonisten, die auch potentiell in vivo bei geringerer Dosis einsetzbar wären.

Die Variation der für die Struktur-Wirkungs-Beziehung bislang als entscheidend angesehenen Fucose- und Neuraminsäurebausteine (B.K.Brandley et al., Glycobiology 1993, 3, 633 und M.Yoshida et.al., Glycoconjugate J. 1993, 10, 3) erbrachte jedoch keine signifikant verbesserten Inhibitionswerte. Allein bei Variation des Glucosaminbausteins (Ersatz von GlcNAc durch Glucose und Azido- sowie Aminogruppen in der 2-Position von GlcNAc) ließ sich eine signifikant erhöhte Affinität an den E-Selektin-Rezeptor erreichen. Beim P-Selektin-Rezeptor wurde hingegen keine verbesserte Bindung erreicht.

Die IC₅₀-Daten dieser Oligosaccharid-Derivate soll für die Hemmung der Adhäsion von HL-60 und U-937 Zellen bei 0.12 mM (gegenüber 1.2-2.0 mM für SLeX) bei E-Selektin liegen. Von Nachteil ist hingegen, daß die Bindung an L- und P-Selektine mit > 5 mM stark beeinträchtigt wird (Dasgupta et al., Posterpräsentation der Firma Glycomed Inc. anläßlich der Tagung in La Jolla in 5/94).

In *The Journal of Biological Chemistry,* vol 269, No 31, pp 19663-19666, 1994 werden Glycyrrhizin-Derivate beschrieben, die in einem zellfreien Assaysystem mit sLeX-Hexasaccharid-ceramid Aktivitäten im IC50-Bereich von 0.5mM bis <2 mM aufweisen. Dabei soll es sich um Mimetika des Sialyl Lewis X handeln, obwohl die sehr komplexen Strukturen nur geringe Ähnlichkeit mit Sialyl Lewis X aufweisen und kein direkter Vergleich der Wirksamkeit mit Sialyl Lewis X selbst gegeben wird.

In der Europäischen Patentanmeldung EP 671 409 werden Malonsäurederivate mit antiadhäsiven Eigenschaften beschrieben. Bei diesen Verbindungen mit guter Wirksamkeit in Zellassays ist die Anbindung einer Malonsäure über einen flexiblen Linker mit einer Pyranose, Furanose oder einem offenkettigen Polyalkohol entscheidend. Zwar stellen diese Verbindungen potentielle Liganden der Selektine dar, doch wird durch die Angaben in dieser Anmeldung nicht belegt bzw. beschrieben, ob diese Verbindungen geeignete Liganden im lebenden Tier bzw. im Menschen darstellen. Die polare, hydrophile und die dianionische Struktur dieser Malonsäuren gemeinsam mit der flexiblen Molekülkette lassen eher ungünstige pharmakologische Eigenschaften, insbesondere eine geringe Bioverfügbarkeit erwarten.

Generell blieben bisher alle Erfolge zur Verbesserung der Bindungsaffinität von SLeX-und SLeA-Derivaten auf den E-Selektinrezeptor beschränkt, denn mit dem P-Selektinrezeptor wurden nur schwache Inhibitionseffekte bei Inhibitorkonzentrationen von ca. 1 mM gefunden (R.M.Nelson et al., J.Clin.Invest. 1993, 91, 1157).

Der Stand der Technik zur Bindungsaffinität modifizierter SLeX/A-Strukturen an Selektine wird in Pharmacochem. Libr. 1993, 20 (Trends in Drug Research), Seiten 33-40 referiert.

Die Aufgabe der vorliegenden Erfindung besteht in der Herstellung neuer Selektin-Liganden, welche eine im Vergleich zu den natürlichen Liganden deutlich stärkere Bindung zu den Rezeptoren aufweisen und sich außerdem leichter als diese synthetisieren lassen.

Die gestellte Aufgabe wird gelöst durch eine Verbindung der Formel I 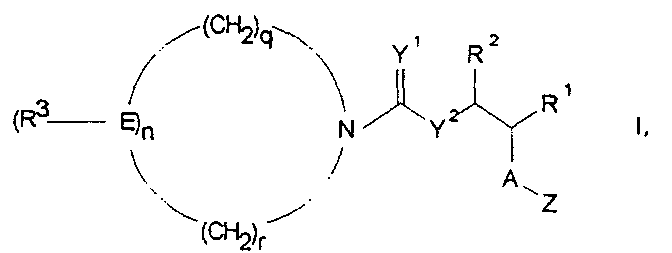 worin bedeuten
- Z: ein Pyranosid, ein über die C6-Position verknüpfter Pyranosylrest, ein über die C6-Position verknüpftes Alkyl-Pyranosid, ein Furanosid, ein über die C5-Position verknüpfter Furanosylrest, ein über die C5-Position verknüpftes Alkyl-Furanosid oder ein Polyalkohol, welcher über eine beliebige Position mit A verknüpft ist,
- A: Sauerstoff, -CH₂- oder Schwefel,
- R¹ und R²: unabhängig voneinander Wasserstoff, -(CH₂)ₘX¹ oder CH₂O(CH₂)ₘX², wobei m eine ganze Zahl von 1 bis 20 bedeutet, oder gemeinsam einen fünf- oder sechsgliedrigen Carbo- oder Heterocyclus mit mindestens einem der Substituenten R⁴, R⁵ oder R⁶,
- E: Stickstoff, Kohlenstoff oder -CH-,
- R³: -(CH₂)ₚCOOH, (-COOH)₂, -(CH₂)ₚCH(COOH)₂, -(CH₂)ₚCNH₂(COOH)₂, -(CH₂)ₚC(CH₂-C₆H₅)(COOH)₂, -CONHC(COOH)₂, wobei p eine ganze Zahl von 0 bis 10 bedeutet, oder 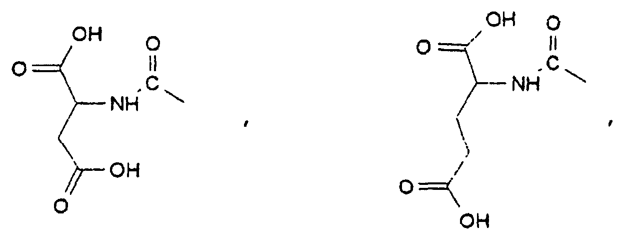 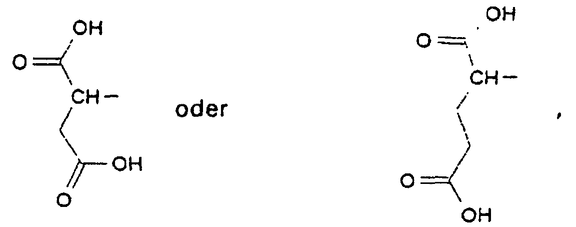
- q und r: unabhängig voneinander eine ganze Zahl von 0 bis 3,
- n: eine ganze Zahl von 1 bis 3 mit der Maßgabe, daß die Summe von q, r und n 4 oder 5 beträgt,
- R⁴, R⁵ und R⁶: unabhängig voneinander H, OH, -O(CH₂)_{w}X³ oder CH₂O(CH₂)_{w}X⁴, wobei w eine ganze Zahl von 1 bis 18 bedeutet,
- Y¹ und Y²: unabhängig voneinander Sauerstoff, -NH- oder Schwefel und
- X¹, X², X³ und X⁴: unabhängig voneinander Wasserstoff, -NH₂, -COOH, -OH, -CH₂OH, CH₂NH₂, -C₁-C₂₀-Alkyl oder -C₆-C₁₀-Aryl.

Vorzugsweise ist die Verbindung der Formel I, dadurch gekennzeichnet, daß R¹ und R² gemeinsam einen Cyclohexanring oder gemeinsam einen Cyclopentanring bilden. Vorzugsweise bedeuten A, Y¹, und Y² in Formel I Sauerstoff.

Besonders bevorzugte Verbindungen gemäß der vorliegenden Erfindung zeichnen sich ferner dadurch aus, daß Z in Formel I ein Pyranosid, vorzugsweise ein L-Fucosid, ein D-Mannosid, L-Rhamnosid, L-Galactosid oder ein L-Mannosid bedeutet.

Besonders geeignet sind auch Verbindungen der Formel I, welche dadurch gekennzeichnet, sind, daß Z ein Furanosid, vorzugsweise ein Ribosid ist.

Weitere bevorzugte Ausgestaltungen der vorliegenden Erfindung zeichnen sich dadurch aus, daß Z in Formel I ein über die C6-Position verknüpfter D-Mannosylrest oder ein ebenso verknüpftes Methyl-D-Mannosid ist. Z in Formel I bedeutet vorzugsweise auch einen L-Threit-1-yl-Rest.

Bevorzugte Ausgestaltungen des aus N (Stickstoff), (CH₂)_{q}, (CH₂)ᵣ und (R³-E)ₙ gebildeten Heterocyclus in Formel I zeichnen sich dadurch aus, daß
n 1 und q und r 2,
n und r 1 und q 3 oder
n 1, q 0 und r 3 bedeuten. Der Substituent R³ in Formel I bedeutet
vorzugsweise -(CH₂)ₚCOOH, wobei p 0 ist, oder -(CH₂)ₚCH(COOH)₂, wobei p 1 ist, wobei in beiden Fällen die Variable E bevorzugt -CH- bedeutet. Vorzugsweise bedeutet R³ auch (-COOH)₂, wobei E für Kohlenstoff steht.

Nachfolgend sind Beispiele für Verbindungen mit den genannten, bevorzugten Eigenschaften dargestellt.
1. Eine Verbindung der Formel I, die sich dadurch auszeichnet, daß
   - Z: ein Pyranosid, beispielsweise ein L-Fucosid und
   - A, Y¹ und Y²: Sauerstoff bedeuten,
   - R¹ und R²: gemeinsam einen Cyclohexanring bilden,
   - R³: (CH₂)ₚCOOH, E -CH-,
   - n: 1,
   - q und r: 2 und p 0 bedeuten, beispielsweise 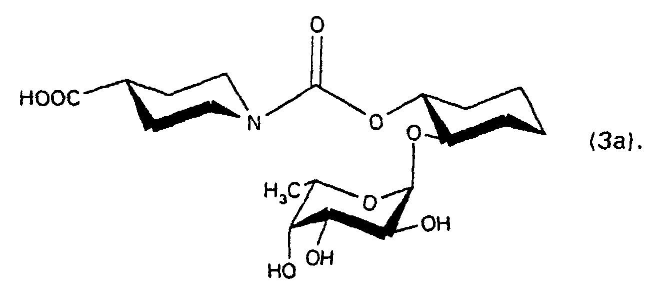
2. Eine Verbindung der Formel I, die sich dadurch auszeichnet, daß
   - Z: ein Pyranosid, beispielsweise ein L-Fucosid und
   - A, Y¹ und Y²: Sauerstoff bedeuten,
   - R¹ und R²: gemeinsam einen Cyclohexanring bilden,
   - R³: (CH₂)ₚCH(COOH)₂, E -CH-,
   - n und r: 1,
   - q: 3 und
   - p: 1 bedeuten, z.B. 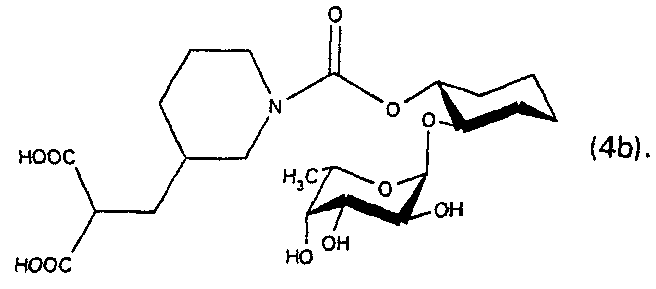
3. Eine Verbindung der Formel I, die sich dadurch auszeichnet, daß
   - Z: ein Pyranosid, beispielsweiseein L-Fucosid und
   - A, Y¹ und Y²: Sauerstoff bedeuten, wobei
   - R¹ und R²: gemeinsam einen Cyclohexanring bilden,
   - R³: (COOH)₂, E Kohlenstoff,
   - n: 1 und
   - q und r: 2 bedeuten, beispielsweise 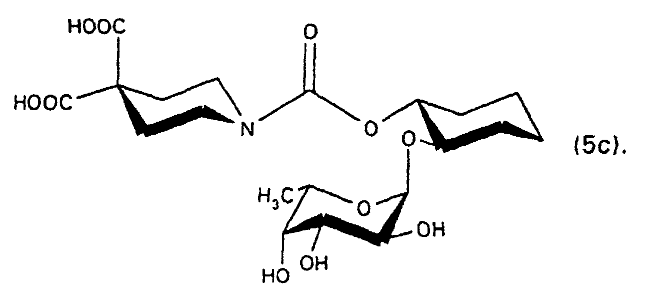
4. Eine Verbindung der Formel I, die sich dadurch auszeichnet, daß
   - Z: ein Pyranosid, beispielsweise ein L-Rhamnosid, und
   - A, Y¹ und Y²: Sauerstoff bedeuten,
   - R¹ und R²: gemeinsam einen Cyclohexanring bilden,
   - R³: (CH₂)ₚCOOH, E -CH-, p 0
   - n: 1 und
   - q und r: 2 bedeuten, beispielsweise 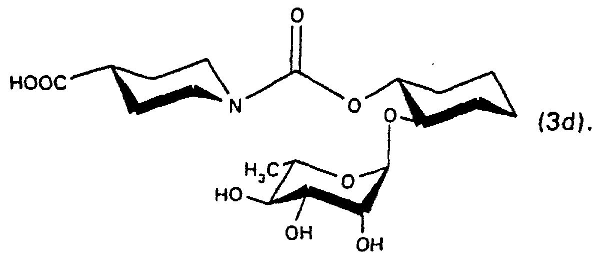
5. Eine Verbindung der Formel I, die sich dadurch auszeichnet, daß
   - Z: ein Pyranosid, beispielsweise ein L-Fucosid, und
   - A, Y¹ und Y²: Sauerstoff bedeuten, wobei
   - R¹ und R²: gemeinsam einen Cyclopentanring bilden,
   - R³: (CH₂)ₚCOOH, E-CH-, p 0 und
   - n: 1 und
   - q und r: 2 bedeuten, beispielsweise 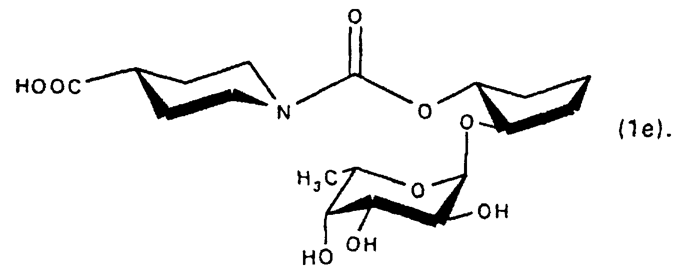
6. Eine Verbindung der Formel 1, die sich dadurch auszeichnet, daß
   - Z: ein Pyranosid, beispielsweise ein L-Fucosid, und
   - A, Y¹ und Y²: Sauerstoff bedeuten, wobei
   - R¹ und R²: gemeinsam einen Cyclohexanring bilden und
   - R³: (CH₂)ₚCH(COOH)₂, p 1, E -CH- und
   - n: 1
   - q: 0 und
   - r: 3 bedeuten, beispielsweise

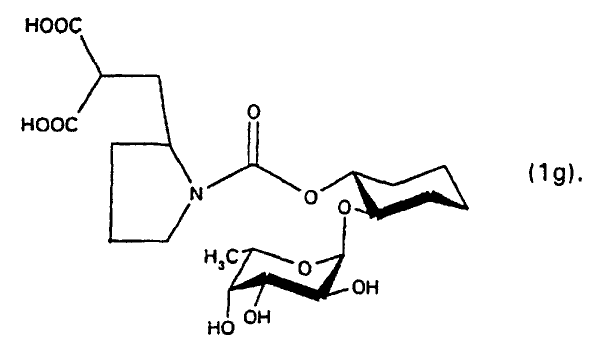

   Weitere Beispiele sind Verbindungen der Formel I, welche sich dadurch auszeichnen, daß
   - R¹ und R²: gemeinsam einen Cyclohexanring bilden und
   - A, Y¹ und Y²: Sauerstoff bedeuten, wobei
   - n: 1,
   - q und r: 2,
   - R³: (CH₂)ₚCOOH, E -CH-, p 0 bedeuten und
7. Z einen L-Threit-1-yl-Rest darstellt, z.B. 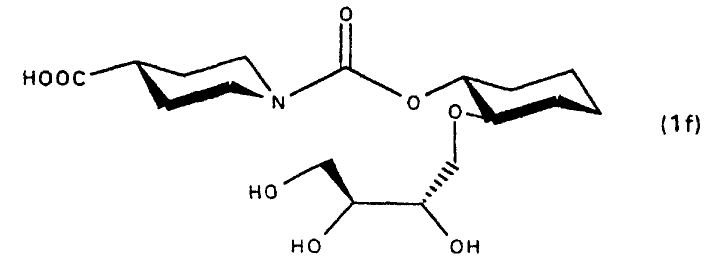 oder
   - Z: ein über die C6-Position verknpüftes Alkylpyranosid,
8. beispielsweise ein Methyl-α-D-mannopyranosid darstellt, 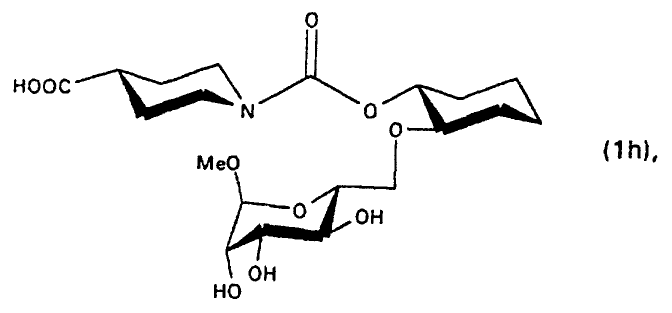 oder
9. beispielsweise ein Methyl-β-D-galactopyranosid 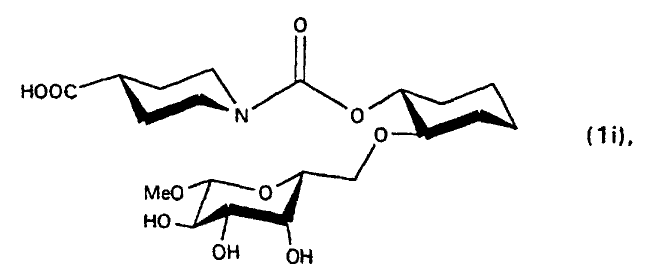 oder
   - Z: ein über die C₆-Position verknüpfter Pyranosylrest, beispielsweise ein Galactosylrest, darstellt, z.B.

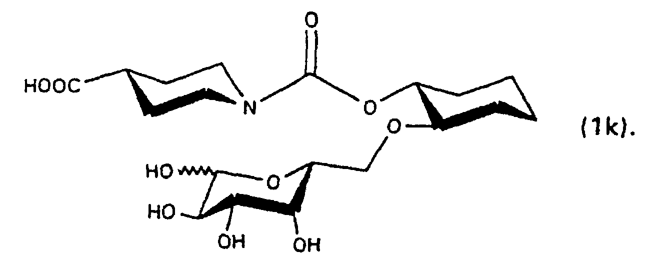

Die eingangs gestellte Aufgabe wird ferner gelöst durch ein Verfahren zur Herstellung einer Verbindung der Formel I, welches sich dadurch auszeichnet, daß man zunächst unter O- oder S-Glycosylierung, Alkylierung oder C-C-Verknüpfung einer funktionellen Gruppe eines Akzeptors der Formel II, 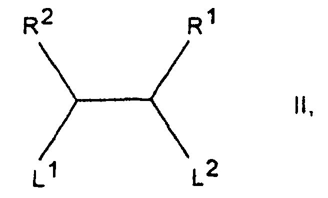 aufweisend mindestens zwei benachbarte funktionelle Gruppen L¹ und L² sowie aufweisend die Substituenten R¹ und R², mittels eines Äquivalents eines mit einer aktivierenden Gruppe L³ und gegebenenfalls mit Schutzgruppen versehenen Donors der Formel III

Z ― L³ III,

Zwischenverbindung IV 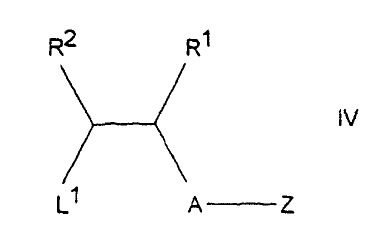 herstellt, wonach man unter Umsetzung mit Reagenz der Formel V, 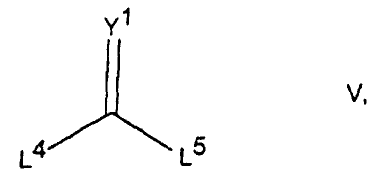 worin L⁴ und L⁵ die Bedeutung von Abgangsgruppen haben, zu der aktivierten Verbindung VI 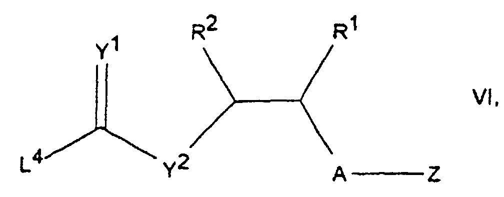 gelangt, welche durch Umsetzung mit einem einfach oder mehrfach carboxylierten cyclischen Amin oder einer geeigneten Vorstufe davon und gegebenenfalls nach Cyclisierung oder Carboxylierung sowie nach Abspaltung von Schutzgruppen zu der Verbindung der Formel I überführt wird, wobei die Variablen R¹, R², Y¹, Y², A und Z die genannten Bedeutungen haben.

Die erfindungsgemäßen Verbindungen mit der allgemeinen Formel I lassen sich ausgehend von käuflichen Komponenten mit mindestens 2 benachbarten funktionellen Gruppen (Akzeptor II), wie z.B. (1R,2R)-trans-1,2-Cyclohexandiol oder Tri-O-acetyl-D-glucal, herstellen. Bei diesen Verbindungen kann beispielsweise (im ersten Fall) durch Glycosylierung mit einem Kohlenhydrat-Donor (z.B. Trichloracetimidat, Ethylthioglycosid etc.) oder Alkylierung mit einem aktivierten Polyalkohol (z.B. Tosylat von 1,2,3-Tri-O-benzyl-L-threitol) die erste der beiden benachbarten funktionellen Gruppen (z.B. eine Hydroxylgruppe) umgesetzt werden (Zwischenverbindung IV). Die noch verbleibende funktionelle Gruppe (L¹, z.B. ebenfalls eine Hydroxylgruppe) kann nun beispielsweise mit Chlorameisensäurenitrophenylester (Reagenz der Formel V mit den Abgangsgruppen Cl und O-C₆H₄-NO₂) zum Nitrophenylcarbamat (Verbindung VI) umgesetzt werden, welches noch im gleichen Reaktionsgefäß mit einem cyclischen Amin (z.B. Piperidin-4-carbonsäure-ethylester) oder einer geeigneten Vorstufe [z.B. 3-(Hydroxymethyl)-piperidin] zu Verbindungen der Formel I bzw. zu deren Vorstufen (siehe Beispiel 4b) reagiert.

Die erfindungsgemäßen Verbindungen der Formel können trotz ihrer wesentlich geringeren Molmasse als Sialyl Lewis X eine höhere Affinität zu den natürlichen Rezeptoren, beispielsweise zu E- und P- Selectin, als dieses haben. Dies kann anhand der nachfolgend beschriebenen Zelladhäsionsassays nachgewiesen werden.

Primärassays zur Untersuchung der Wirkung der Verbindungen gemäß der vorliegenden Erfindung auf die Zellanheftung an rekombinante, lösliche Selektin-Fusionsproteine.

Um die Wirksamkeit der erfindungsgemäßen Verbindungen auf die Interaktion zwischen den E- und P-Selektinen (alte Nomeklatur ELAM-1 bzw. GMP-140) mit ihren Liganden zu testen, wird ein Assay verwendet, der jeweils nur für eine dieser Interaktionen spezifisch ist. Die Liganden werden in ihrer natürlichen Form als Oberflächenstrukturen auf promyelozytischen HL60 Zellen angeboten. Da HL60 Zellen Liganden und Adhäsionsmoleküle unterschiedlichster Spezifität aufweisen, kann die gewünschte Spezifität des Assays nur über den Bindungspartner erbracht werden. Als Bindungspartner wurden gentechnisch hergestellte lösliche Fusionsproteine aus der jeweils extrazytoplasmatischen Domäne von E- bzw. P-Selektin und der konstanten Region eines humanen Immunglobulins der Subklasse IgG1 verwendet.

### Herstellung von L-Selektin-IgGI

Zur Herstellung von löslichem L-Selektin-IgG1 Fusionsprotein wurde das von Walz et al., 1990 publizierte genetische Konstrukt "ELAM-Rg" verwendet.

Zur Expression wurde die Plasmid DNA in COS-7 Zellen (ATCC) mittels DEAE-Dextran transfiziert (Molekularbiologische Methoden: siehe Ausubel, F. M., Brent, R., Kingston, R. E., Moore, D. D., Seidman, J. G., Struhl, K. und Smith, J. A. 1990. Current Protocols in Molecular Biology, John Wiley, New York). Sieben Tage nach der Transfection wird der Kulturüberstand gewonnen, durch Zentrifugation von Zellen und Zellfragmenten befreit und auf 25 mM Hepes pH 7,0, 0,3 mM PMSF, 0,02 % Natriumazid gebracht und bei +4°C aufgehoben. (Walz, G., Aruffo, A., Kolanus, W., Bevilacqua, M. und Seed, B. 1990. Recognition by ELAM-1 of the sialyl-Lex determinant on myeloid and tumor cells. Science 250, 1132-1135.)

### Herstellung von P-Selektin-IgG1

Zur Herstellung des löslichen P-Selektin-IgG1 Fusionsproteins wird das von Aruffo et al., 1991 publizierte genetische Konstrukt "CD62Rg" verwendet. Die weitere Vorgehensweise entspricht der unter A1 dargestellten Herstellung von L-Selektin-IgG1.

Aruffo, A., Kolanus, W., Walz, G., Fredman, P. und Seed, B. 1991. CD62/P-Selectin recognition of myeloid and tumor cell sulfatides. Cell 67, 35-44.

### Herstellung von CD4-IgG1

Zur Herstellung des löslichen CD4-IgG1 Fusionsproteins wird das von Zettlemeissl et al., 1990 publizierte genetische Konstrukt "CD4:IgG1 hinge" verwendet. Die weitere Vorgehensweise entspricht der unter A1 dargestellten Herstellung von L-Selektin-IgG1. (Zettelmeissl, G., Gregersen, J.-P., Duport, J. M., Mehdi, S., Reiner, G. und Seed, B. 1990. Expression and characterization of human CD4: Immunoglobulin Fusion Proteins. DNA and Cell Biology 9, 347-353.)

Durchführung des HL60 Zelladhäsionsassays auf rekombinanten, löslichen Adhäsionsmolekülen
1. 96er Mikrotitertestplatten (Nunc Maxisorb) werden mit 100 µl eines in 50 mM Tris pH 9,5 verdünnten (1 + 100) Ziege anti human IgG Antikörpers (Sigma) 2 Std. bei Raumtemperatur inkubiert. Nach Entfernen der Antikörperlösung wird einmal mit PBS gewaschen.
2. 150 µl des Blockierungspuffers werden für 1 Std. bei Raumtemperatur in den Näpfchen belassen. Die Zusammensetzung des Blockierungspuffers ist: 0,1 % Gelatine, 1 % BSA, 5 % Kalbserum, 0,2 mM PMSF, 0,02 % Natriumazid. Nach Entfernen des Blockierungspuffers wird einmal mit PBS gewaschen.
3. In die Näpfchen werden je 100 µl Zellkulturüberstand von entsprechend transfektierten und exprimierenden COS-Zellen pipettiert. Die Inkubation erfolgt 2 Std. bei Raumtemperatur. Nach Entfernen des Zellkulturüberstandes wird einmal mit PBS gewaschen.
4. In die Näpfchen werden 20 µl Bindungspuffer gegeben. Der Bindungspuffer hat die Zusammensetzung: 50 mM Hepes, pH 7,5; 100 mM NaCl; 1 mg/ml BSA;
   2 mM MgCl₂; 1mM CaCl₂; 3 mM MnCl₂; 0,02 % Natriumazid; 0,2 mM PMSF. Dazu werden 5 µl der Testsubstanz pipettiert, durch Schwenken der Platte vermischt und 10 Min. bei Raumtemperatur inkubiert.
5. 50 ml einer HL60 Zellkultur mit 200.000 Zellen/ml werden 4 Min. bei 350 g zentrifugiert. Das Pellet wird in 10 ml RPMI 1640 resuspendiert und die Zellen erneut zentrifugiert. Zur Markierung der Zellen werden 50 µg BCECF-AM (Molecular Probes) in 5 µl wasserfreiem DMSO aufgelöst; anschließend werden 1,5 ml RPMI 1640 auf die BCECF-AM/DMSO-Lösung gegeben. Mit dieser Lösung werden die Zellen resuspendiert und 30 Min. bei 37°C inkubiert. Nach zweiminütiger Zentrifugation bei 350 g wird das markierte Zellpellet in 11 ml Bindungspuffer resuspendiert und die resuspendierten Zellen in 100 µl Aliquots in die Mikrotiterplatten-Näpfchen verteilt. Die Platte wird 10 Min. bei Raumtemperatur stehen gelassen, um die Zellen auf den Boden der Testplatte sedimentieren zu lassen. Dabei haben die Zellen Gelegenheit an das beschichtete Plastik zu adhärieren.
6. Zum Abstoppen des Tests wird die Mikrotiterplatte im 45° Winkel gänzlich in den Stoppuffer getaucht (25 mM Tris, pH 7,5; 125 mM NaCI; 0,1 % BSA; 2 mM MgCl₂; 1 mM CaCl₂; 3 mM MnCl2; 0,02 % Natriumazid). Durch Invertierung wird der Stoppuffer aus den Näpfchen entfernt und die Prozedur noch zweimal wiederholt.
7. Die Messung der in den Näpfchen festhaftenden, BCECF-AM-markierten Zellen erfolgt in einem Cytofluorimeter (Millipore), bei einer Empfindlichkeitseinstellung von 4, einer Anregungswellenlänge von 485/22 nm und einer Emissionswellenlänge von 530/25 nm.

### Ergebnisse:

### IC 50-Werte für E-Selektin [mM], in runden Klammern für P-Selektin [mM]:

N-Carbonyl-4-carboxypiperidyl-(1→2)-[(α-L-fucopyranosyl)-(1→1)]-(1R,2R)-trans-1,2-cyclohexandiol (3a):
   größer als 2 (größer als 2)
[N-Carbonyl-(1,1-dicarboxyl-ethyl)-(2→3)-piperidyl]-(1→2)-[(α-L-fucopyranosyl)-(1→1)]-(1R,2R)-trans-1,2-cyclohexandiol (4b):
   größer als 2 (größer als 2)
N-Carbonyl-4-carboxypiperidyl-(1→2)-[(α-L-fucopyranosyl)-(1→1)]-(1R, 2R)-trans-1,2-cyclopentandiol (1e):
   3,7 (2,85)
N-Carbonyl-4,4-dicarboxypiperidyl-(1→2)-[(-α-L-fucopyranosyl)-(1→1)]-(1R,2R)-trans-1,2-cyclohexandiol (3e):
   1,6 (7,5)

### Leukozyten-Adhäsion - Prüfung der Wirksamkeit der erfindungsgemäßen Verbindungen in vivo (Intravitalmikroskopie an der Ratte):

Bei entzündlichen Prozessen und anderen, die Zytokine aktivierenden Zuständen spielt die Gewebszerstörung durch einwandernde oder die Mikrozirkulation blockierende Leukozyten eine entscheidende Rolle. Die erste und für den weiteren Krankheitsprozeß entscheidende Phase ist die Aktivierung von Leukozyten innerhalb der Blutbahn, insbesondere im prä- und postkapillären Bereich. Dabei kommt es, nachdem die Leukozyten den Axialstrom des Blutes verlassen haben, zu einem ersten Anheften der Leukozyten an der Gefäßinnenwand, d.h. am Gefäßendothel. Alle darauf folgenden Leukozyteneffekte, d.h. die aktive Durchwanderung durch die Gefäßwand und die anschließende orientierte Wanderung im Gewebe, sind Folgereaktionen (Harlan, J.M., Leukocyte-endothelial interaction, Blood 65, 513-525, 1985).

Diese rezeptorvermittelte Interaktion von Leukozyten und Endothelzellen wird als ein initiales Zeichen des Entzündungsprozesses angesehen. Neben den schon physiologisch exprimierten Adhäsionsmolekülen kommt es unter der Einwirkung von Entzündungsmediatoren (Leukotriene, PAF) und Zytokinen (TNF-alpha, Interleukinen) zur zeitlich gestuften, massiven Expression von Adhäsionsmolekülen auf den Zellen. Sie werden derzeit in drei Gruppen eingeteilt: 1. Immunglobulin-Gensuperfamilie, 2. Integrine und 3. Selektine. Während die Adhäsion zwischen Molekülen der Ig-Gensuperfamilie und den Protein-Protein-Bindungen abläuft, stehen bei der Kooperation zwischen Selektinen Lektin-Kohlehydrat-Bindungen im Vordergrund (Springer, T.A., Adhesion receptors of the immune system. Nature 346, 425-434, 1990; Huges, G., Cell adhesion molecules - the key to an universal panacea, Scrips Magazine 6, 30-33, 1993; Springer, T.A., Traffic signals for lymphocyte recirculation and leukocyte emigration; The multistep paradigm. Cell 76, 301-314, 1994).

### Methode:

Die induzierte Adhäsion von Leukozyten wird mit einer intravitalmikroskopischen Untersuchungstechnik im Mesenterium der Ratte quantifiziert (Atherton A. and Born G.V.R., Quantitative investigations of the adhesiveness of circulating polymorphnuclear leukocytes to blood vessel walls. J. Physiol. 222, 447-474, 1972; Seiffge, D. Methoden zur Untersuchung der Rezeptor-vermittelten Interaktion zwischen Leukozyten und Endothelzellen im Entzündungsgeschehen, in: Ersatz- und Ergänzungsmethoden zu Tierversuchen in der biomedizinischen Forschung, Schöffl, H. et al., (Hrsg.) Springer, 1995 (im Druck)). Unter Inhalations-Äthernarkose wird eine Dauernarkose durch intramuskuläre Injektion von Urethan (1,25 mg/kg KG) eingeleitet. Nach Freipräparation von Gefäßen (V. femoralis zur Injektion von Substanzen und A. carotis zur Blutdruckmessung) werden Katheter in diese eingebunden. Danach wird das entsprechende transparente Gewebe (Mesenterium) nach den in der Literatur bekannten Standardmethoden freigelegt und auf dem Mikroskoptisch ausgelagert und mit 37°C warmen Paraffinöl überschichtet (Menger, M.D. and Lehr, H., A. Scope and perspetives of intravital microscopy-bridge over from in vitro to in vivo, Immunology Today 14, 519-522, 1993). Die Applikation der Testsubstanz erfolgt i.v. am Tier (10mg/kg). Die experimentelle Erhöhung der Blutzell-Adhäsion wird durch systemische Verabreichung von Lipopolysaccharid (LPS, 15 mg/kg) 15 Minuten nach Applikation aus Testsubstanz durch Zytokin-Aktivierung ausgelöst (Foster S.J., Mc Cormick L.M., Ntolosi B.A. and Campbell D., Production of TNF-alpha by LPS-stimulated murine, rat and human blood and its pharmacological modulation, Agents and Actions 38, C77-C79, 1993, 18.01.1995). Die dadurch verursachte erhöhte Adhäsion von Leukozyten am Endothel wird direkt vitalmikroskopisch oder mit Hilfe von Fluoreszenzfarbstoffen quantifiziert. Alle Meßvorgänge werden per Videokamera aufgenommen und auf einem Videorekorder gespeichert. Über einen Zeitraum von 60 Minuten wird alle 10 Minuten die Anzahl der rollenden Leukozyten (d.h. alle sichtbar rollenden Leukozyten, die langsamer als die strömenden Erythrozyten sind) und die Anzahl an haftenden Leukozyten am Endothel (Verweildauer länger als 5 Sekunden) erfaßt. Nach Beendigung des Versuches werden die narkotisierten Tiere schmerzfrei durch systemische Injektion von T61 exzitationsfrei eingeschläfert. Zur Auswertung werden die Ergebnisse jeweils von 8 behandelten mit 8 unbehandelten Tieren (Kontrollgruppe) verglichen (Angabe der Ergebnisse in Prozenten).

### Ergebnisse:

- 3a:: Dosis: 10 mg/kg; Applikation: i.v.; Spezies: SPRD (m); Gewicht in g: 298 +/- 17,72; Anzahl der Gefäße: 15; Gefäßdurchmesser in µm 24 +/- 5; Leukocyten in 10³/mm³: 7,7 +/- 2,66; Fibrinogen in mg/100 ml: 135 +/- 21,71; Inhibition: 81%.
Dosis: 5 mg/kg; Applikation: i.v.; Spezies: SPRD (m); Gewicht in g: 306 +/- 6.65; Anzahl der Gefäße: 16; Gefäßdurchmesser in µm 27 +/- 4; Leukocyten in 10³/mm³: 7,5 +/- 1,93; Fibrinogen in mg/100 ml: 101 +/- 5,75; Inhibition: 69%.
Dosis: 1 mg/kg; Applikation: i.v.; Spezies: SPRD (m); Gewicht in g: 333 +/- 21,6; Anzahl der Gefäße: 16; Gefäßdurchmesser in µm 25 +/- 4,1; Leukocyten in 10³/mm³; 7,3 +/- 1,4; Fibrinogen in mg/100 ml: 117 +/- 15,8; Inhibition: 64%.

Reperfusionsmodell zur Untersuchug des Einflusses der Adhäsion von Neutrophilen im Verlauf der Ischämie/Reperfusion am offenen Kaninchen-Herzen.

Die Herzen werden bei konstantem Druck nach der Langendorff-Technik mit Nährlösung sowie mit/ohne Leukozyten bzw. Wirkstoff perfundiert. Danach wird eine Ischämie durch Unterbinden der linken Koronarartherie (30 min) hervorgerufen. Nach Reperfusion (30 min) wird die Leukozytenakkumulierung histologisch ausgewertet. Im Versuchsverlauf werden außerdem an 256 Elektroden Potentiale und Arrhythmien gemessen (Gesamtversuchsdauer ca. 90 min). Bei 6 von 7 unbehandelten mit Leukozyten perfundierten Herzen treten ausgeprägte Arrhythmien aufgrund der Leukozyteninfiltration auf, während die mit einem Wirkstoff (RGDS-Peptide, Chondroitinsulfat) behandelten Herzen verminderte Leukozyten Akkumulierung und Arrhythmien entwickeln. Die untersuchte Verbindung 3a war im Bereich von ca. 1 µM hochwirksam (starke Verminderung der Arrhythmien).

Die Verbindungen gemäß der vorliegenden Erfindung sowie deren physiologisch verträglichen Salze eignen sich aufgrund ihrer wertvollen pharmakologischen Eigenschaften sehr gut zur Anwendung als Heilmittel bei Säugern, insbesondere dem Menschen.

Die vorliegende Erfindung betrifft daher ferner ein Arzneimittel enthaltend wenigstens eine Verbindung gemäß Formel I sowie deren Verwendung für die Herstellung eines Arzneimittels zur Therapie oder Prophylaxe von Krankheiten, welche mit einer übermäßigen, durch Selektinrezeptoren vermittelten Zelladhäsion in dem von der Krankheit betroffenen Gewebe einhergehen, beispielsweise Rheuma, Herz-Kreislauf-Erkrankungen, wie Reperfusionsverletzungen, Ischämie oder Herzinfarkt.

Die Arzneimittel eignen sich im besonderen zur Behandlung von akuten und chronischen Entzündungen, die sich pathophysiologisch durch eine Störung der Zellzirkulation, beispielsweise von Lymphozyten, Monozyten und neutrophilen Granulozyten, kennzeichnen lassen. Hierzu zählen Autoimmunerkrankungen wie akute Polyarthritis, rheumatoide Arthritis und Insulin-abhängige Diabetes (Diabetes mellitus IDDM) akute und chronische Transplantatabstoßungen, Schocklunge (ARDS, adult respiratory distress syndrome), entzündliche und allergische Hauterkrankungen wie beispielsweise Psoriasis und Kontakt-Ekzeme, Herz-Kreislauf-Erkrankungen wie Myokardinfarkt, Reperfusionsverletzungen nach Thrombolyse , Angioplastie oder By-Pass-Operationen, septischer Schock und systemischer Schock. Eine weitere potentielle Indikation ist die Behandlung metastasierender Tumoren, denn Tumorzellen tragen Oberflächenantigene, die sowohl Sialyl-Lewis-X als auch Sialyl-Lewis-A-Strukturen als Erkennungsepitope besitzen. Darüberhinaus können diese Arzneimittel, die stabil im sauren Milieu des Magens sind, zur antiadhäsiven Therapie von Helicobacter pylori und verwandten Mikroorganismen ggf. auch in Kombination mit Antibiotika eingesetzt werden. Ferner ist mit Hilfe dieser Arzneimittel eine Therapie der cerebralen Form der Malaria denkbar.

Die erfindungsgemäßen Arzneimittel werden im allgemeinen intravenös, oral oder parenteral oder als Implantate verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierte Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z.B. Magnesiumcarbonat, Titandioxid, Laktose, Mannit und andere Zucker, Talkum, Milcheiweiß, Gelatine, Stärke, Vitamine, Cellulose und ihre Derivate, tierische und pflanzliche Öle, Polyethylenglykole und Lösungsmittel, wie etwa steriles Wasser, Alkohole, Glycerin und mehrwertige Alkohole genannt.

Vorzugsweise werden die pharmazeutischen Präparate in Dosierungseinheiten hergestellt und verabreicht. Feste Dosierungseinheiten sind Tabletten, Kapseln und Suppositorien.

Für die Behandlung eines Patienten sind je nach Wirksamkeit der Verbindung, Art der Applikation, Art und Schwere der Erkrankung, Alter und Körpergewicht des Patienten, unterschiedliche Tagesdosen notwendig. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleiner Dosierungseinheiten als auch durch Mehrfachgabe unterteilten Dosen in bestimmten Intervallen erfolgen. Die zu verabreichende Tagesdosis kann außerdem von der Anzahl der während des Krankheitsverlaufs exprimierten Rezeptoren abhängig sein. Es ist vorstellbar, daß im Anfangsstadium der Krankheit nur wenige Rezeptoren auf der Zelloberfläche exprimiert werden und demzufolge die zu verabreichende Tagesdosis geringer ist als bei stark erkrankten Patienten.

Die erfindungsgemäßen Arzneimittel werden dadurch hergestellt, daß man eine Verbindunmg gemäß der vorliegenden Erfindung mit üblichen Träger- sowie gegebenenfalls Zusatz- und/oder Hilfsstoffen in die bzw. eine geeignete Darreichungsform bringt.

Ferner ist die Verwendung von Verbindungen gemäß der Formel I für die Herstellung eines Mittels zur Diagnose einer Krankheit, welche mit einer übermäßigen, durch Selektinrezeptoren vermittelten Zelladhäsion in dem von der Krankheit betroffenen Gewebe einhergeht, denkbar.

Beispiele für die Herstellung der erfindungsgemäßen Verbindungen:

### Beispiel 1

a) Synthese von [(2,3,4-tri-O-benzyl-α-L-fucopyranosyl)-(1→1)]-(1R, 2R)-trans-1, 2-cyclo-hexandiol (1a):
   Eine Mischung aus (1R,2R)-trans-1,2-cyclohexandiol (2,43 g, 20,9 mmol), Thioethyl- 0-2,3,4-tri-O-benzyl-β-L-fucopyranosid (8,0 g, 16,72 mmol) und Tetrabutylammoniumbromid (2,7 g, 8,36 mmol) in Dichlormethan (200 ml) und DMF (40 ml) wird 1h mit Molekularsieb 4 A gerührt. Anschließend wird Kupfer(II)-bromid (5,6 g, 25,08 mmol) zugegeben. Nach 24 h wird über Kieselgur filtriert, mit gesättigter Natriumhydrogencarbonatlösung und danach mit gesättigter Kochsalzlösung gewaschen. Die organische Phase wird über Magnesiumsulfat getrocknet, im Vakuum eingeengt und mit Hexan/Essigester 3:1 chromatografiert. Ausbeute: 6,8 g (76%).
   ¹H-NMR (300 MHz, CDCl₃): δ = 1.13 (d, 3H, 6-H_{fuc}), 1.21 (m, 4H, 4-H_{cyclohex}, 5-H_{cyclohex}), 1.65, 2.01 (2m, 4H, 3-H_{cyclohex}, 6-H_{cyclohex})
b) Synthese von N-Carbonyl-4-carbethoxypiperidyl-(1→2)-[(2,3,4-tri-O-benzyl-α-L-fucopyranosyl)-(1→1)]-(1R,2R)-trans-1,2-cyclohexandiol (2a):
   Zu einer Lösung aus 1a (8,2 g, 15,39 mmol) in Dichlormethan (164 ml), gibt man bei 0 °C Triethylamin (2,3 ml, 16,9 mmol), DMAP (206 mg, 1,69 mmol) und Chlorameisensäurenitrophenylester (3,1 g, 15,39 mmol). Die Mischung wird über Nacht gerührt und mit N-Ethyldiisopropylamin (4,6 ml, 26,9 mmol) sowie Piperidin-4-carbonsäure-ethylester (4,15 ml, 26,9 mmol) versetzt. Es wird nochmal 18 h gerührt. Zur Aufarbeitung wird mit Dichlormethan (500 ml) verdünnt und mit Wasser (3 x 250 ml) gewaschen. Die organische Phase wird im Vakuum eingeengt und mit Hexan/Essigester 3:1→2:1→1:1 chromatografiert. Ausbeute: 9,3 g (84%).
   ¹H-NMR (300 MHz, CDCl₃): δ = 1.09 (d, 3H, 6-H_{fuc}), 1.25 (t, 3H, OCH₂CH₃), 4.14 (q, 2H, OCH₂CH₃).
c) Synthese von N-Carbonyl-4-carboxypiperidyl-(1→2)-[(α-L-fucopyranosyl)-(1→1)]-(1R,2R)-trans-1,2-cyclohexandiol (3a):
   Eine Mischung aus Verbindung 2a (9,75 g, 13,6 mmol) und Palladiumkohle (10%, 9 g) in Methanol/Dioxan/Eisessig (50:5:2, 570 ml) wird 24 h unter Normaldruck in einer Wasserstoffatmosphäre hydriert. Die Palladiumkohle wird abfiltriert, der Rückstand eingeengt und mit 1 M Natronlauge (100 ml) behandelt. Nach 2 h wird mit Amberlite IR-120 neutralisiert und über RP-Kieselgel (C₁₈ Bakerbond 60 Å) mit Wasser/Methanol 9:1→1:9 gereinigt. Man erhält Verbindung 3a (5,18 g, 91%).
   ¹H-NMR (300 MHz, D₂O): δ = 1.05 (d, 3H, 6-H_{fuc}), 1.56 (m, 2H), 1.78 (m, 3H), 2.00 (m, 1H), 2.45 (m, 1H), 2.80 (m, 2H), 4.50 (m, 1H, 2-H_{cyclohex.}), 4.87 (bs, 1H, 1-H_{fuc}).

### Beispiel 2

a) Synthese von N-Carbonyl-3-[(hydroxymethyl)-piperidyl]-(1→2)-[(2,3,4-tri-O-benzyl-α-L-fucopyranosyl)-(1→1)]-(1R,2R)-trans-1,2-cyclohexandiol (1b):
   Verbindung 1b wird analog zu 3a hergestellt. Dazu wird 1a mit Chlorameisensäurenitrophenylester umgesetzt und anschließend 3-(Hydroxymethyl)-piperidin (Aldrich) zugegeben. Die Aufarbeitung erfolgt wie bei 3a beschrieben.
b) Synthese von N-Carbonyl-3-[(p-toluolsulfonyloxymethyl)-piperidyl]-(1→2)-[(2,3,4-tri-O-benzyl-α-L-fucopyranosyl)-(1→1)]-(1R,2R)-trans-1,2-cyclohexandiol (2b):
   Zu einer eiskalten Lösung von Verbindung 1b (650 mg, 0,97 mmol) in Pyridin (13 ml) gibt man p-Toluolsulfonsäurechlorid (277 mg, 1,45 mmol). Nach 18 h gibt man Dichlormethan (100 ml) dazu und wäscht die organische Phase mit ges. Kochsalzlösung (2 x 50 ml). Die organische Phase wird über Natriumsulfat getrocknet, filtriert und im Vakuum eingeengt. Flashchromatografie (Hexan/Essigester 2:1) liefert verbindung 2b (537 mg, 67%).
   ¹H-NMR (300 MHz, CDCl₃): δ = 1.06 (d, 3H, 6-H_{fuc}), 2.04 (s, 3 H, CH₃).
b) Synthese von [N-Carbonyl-(1,1-dicarbmethoxy-ethyl)-(2→3)-piperidyl]-(1→2)-[(α-L-fucopyranosyl)-(1→1)]-(1R,2R)-trans-1,2-cyclohexandiol (3b):
   Eine Mischung aus Verbindung 2b (460 mg, 556 µm), Malonsäuredimethylester (18 ml), Kaliumcarbonat (1,07 g) und Dibenzo-18-crown-6 (264 mg) wird 4 h bei 100 °C gerührt. Zur Aufarbeitung wird mit Dichlormethan (460 ml) verdünnt und die organische Phase abwechselnd mit Wasser und Trockeneis behandelt bis das Waschwasser neutral reagiert. Die organische Phase wird über Natriumsulfat getrocknet und im Hochvakuum bei 80 °C eingengt. Chromatografie (Hexan/Essigester 2:1) liefert Verbindung 3b (367 mg, 84%).
   ¹H-NMR (300 MHz, CDCl₃): δ = 1.08 (d, 3H, 6-H_{fuc}), 3.72 (2 s, 6H, 2 COOCH₃).
c) Synthese von [N-Carbonyl-(1,1-dicarboxy-ethyl)-(2→3)-piperidyl]-(1→2)-[(α-L-fucopyranosyl)-(1→1)]-(1R,2R)-trans-1,2-cyclohexandiol (4b):
   Die Entschützung von 4b verläuft wie unter 3a beschrieben.
   ¹H-NMR (300 MHz, D₂O): δ = 1.09 (d, 3H, 6-H_{fuc}), 4.54 (m, 1H, 2-H_{cyclohex.}), 4.91 (bs, 1H, 1-H_{fuc}).

### Beispiel 3

a) Synthese von 2-Brom-N-(2-bromethyl)-N-carbobenzoxy-ethanamin (1c):
   Zu einer eiskalten Lösung aus Bis-(2-bromethyl)-amin Hydrobromid (4,5g, 14,4 mmol) in Wasser (25 ml) gibt man unter kräftigen Rühren Chlorameisensäure-benzylester (1,97 ml, 13,8 mmol) und 1 M Natronlauge bis der pH-Wert gerade basisch wird (ca. 24 ml). Es wird mit 1 M Salzsäure (2 ml) angesäuert und mit Ether (3 x 40 ml) extrahiert. Die organische Phase wird mit Natriumhydrogencarbonat-Lösung und Wasser gewaschen, über Magnesiumsulfat getrocknet, eingeengt und der Rückstand chromatografiert (Hexan/Essigester 5:1→4:1). Man erhält Verbindung 1c (4,1 g, 81%).
   ¹H-NMR (300 MHz, CDCl₃): δ = 3.43, 3.53 [2 m, 4H, N(CH₂-CH₂Br)₂], 3.73 [m, 4H, N(CH₂-CH₂Br)₂], 5.17 (s, 2H, CH₂Ph), 7.36 (m, 5H, Ph).
b) Synthese von N-Carbobenzoxy-4,4-dicarbethoxypiperidin (2c):
   Zu einer Lösung aus 1c (1,1 g, 3 mmol) in Dimethylformamid (1 ml) gibt man Malonsäurediethylester (303 µl, 2mmol) und erwärmt auf 50 °C. Nach Zugabe von Natriumhydrid (120 mg, 5 mmol) wird noch 12 h gerührt. Die Mischung wird im Hochvakuum eingeengt und mit Hexan/Essigester 9:2→7:2 chromatografiert. Ausbeute 0,5 g (69%).
   ¹H-NMR (300 MHz, CDCl₃): δ = 1.25 (t, 6H, 2 CH₃), 2.08 (m, 4H, C-CH₂-CH₂N), 3.52 (m, 4H, C-CH₂-CH₂N), 4.20 (q, 4H, 2 OCH₂CH₃), 5.12 (s, 2H, CH₂Ph), 7.35 (m, 5H, Ph).
c) Synthese von 4,4-Dicarbethoxypiperidin (3c):
   Eine Mischung aus 2c (778 mg, 2,14 mmol) und Palladiumkohle (78 mg) in Methanol (10 ml) wird 1h unter einer Wasserstoffatmosphäre hydriert. Zur Aufarbeitung wird filtriert und eingeengt. 3c (485 mg, 99%) wird roh in die nächste Stufe eingesetzt.
   ¹H-NMR (300 MHz, CDCl₃): δ = 1.26 (t, 6H, 2 CH₃), 2.06 (m, 4H, C-CH₂-CH₂N), 2.87 (m, 4H, C-CH₂-CH₂N), 4.20 (q, 4H, 2 OCH₂CH₃).
d) Synthese von N-Carbonyl-4,4-dicarbethoxypiperidyl-(1→2)-[(2,3,4-tri-O-benzyl-α-L-fucopyranosyl)-(1→1)]-(1R,2R)-trans-1,2-cyclohexandiol (4c):
   Verbindung 4c wird analog zu 2a aus 1a und 3c synthetisiert.
   ¹H-NMR (300 MHz, CDCl₃): δ = 1.09 (d, 3H, 6-H_{fuc}), 1.27 (2t, 6H, 2 CH₃), 2.02 (m, 4H, C-CH₂-CH₂N), 4.2 (2q, 4H, 2 OCH₂CH₃), 7.3 (m, 15 H, 3 Ph).
e) Synthese von N-Carbonyl-4,4-dicarboxypiperidyl-(1→2)-[(-α-Lfucopyranosyl)-(1→1)]-(1R,2R)-trans-1,2-cyclohexandiol (5c):
   Verbindung 4d wird analog zu 3a entschützt.
   ¹H-NMR (300 MHz, D₂O): δ = 0.95 (d, 3H, 6-H_{fuc}), 1.46 (m, 2H), 1.74 (m, 4H), 1.90 (m, 1H), 3.69 (q, 1H, 5-H_{fuc}), 4.41 (m, 1H, 2-H_{cyclohex.}), 4.78 (bs, 1H, 1-H_{fuc}).

### Beispiel 4

a) Synthese von [(2,3,4-tri-O-Acetyl-α-L-rhamnopyranosyl)-(1→1)]-(1R,2R)-trans-1,2-cyclohexandiol (1d):
   Zu einer Mischung aus (1R,2R)-trans-1,2-cyclohexandiol (401 mg, 3,5 mmol) und O-(2,3,4-Tri-O-acetyl-L-rhamnopyranosyl)-trichloracetimidat (1,0 g, 2,3 mmol) in Dichlormethan (25 ml) gibt man tropfenweise eine 0,1 M Trimethylsilyltrifluormethansulfonat- lösung (0,23 mmol). Nach 20 min wird mit Natriumhydrogencarbonat (200 mg) abgebrochen, filtriert, eingengt und der Rückstand mit Hexan/Essigester 2:1 chromatografiert. Ausbeute: 640 mg (72%).
   ¹H-NMR (300 MHz, CDCl₃): δ = 1.24 (d, 3H, 6-Hᵣₕₐₘ), 2.00, 2.05, 2.16 (3s, 9H, 3 OAc), 4.92 (d, 1H, 1-Hᵣₕₐₘ), 5.08 (dd, 1H, 4-Hᵣₕₐₘ), 5.21 (dd, 1H, 2-Hᵣₕₐₘ), 5.32 (dd, 1H, 3-Hᵣₕₐₘ).
b) Synthese von N-Carbonyl-4-carbethoxypiperidyl-(1→2)-[(2,3,4-tri-O-acetyl-α-L-rhamnopyranosyl)-(1→1)]-(1R,2R)-trans-1,2-cyclohexandiol (2d):
   Verbindung 2d wird analog zu 2a synthetisiert.
c) Synthese von N-Carbonyl-4-carboxypiperidyl-(1→2)-[(α-L-rhamnopyranosyl)-(1→1)]-(1R,2R)-trans-1,2-cyclohexandiol (3d):
   Zu einer Lösung von Verbindung 2d (425 mg, 744 µmol) in Methanol (30 ml) gibt man eine 1M Natriummethanolatlösung (1,05 ml). Nach 1 h wird mit Amberlite IR-120 neutralisiert, filtriert und eingeengt. Zum Rückstand gibt man 1M Natronlauge (10 ml). Nach 1h wird erneut mit Amberlite IR-120 neutralisiert, filtriert und eingeengt. Der Rückstand wird wie bei 3a beschrieben gereinigt. Ausbeute: 258 mg (83%).
d) SynthesevonN-Carbonyl-4-carboxypiperidyl-(1→2)-[(α-L-fucopyranosyl)-(1→1)]-(1R,2R)-trans-1,2-cyclopentandiol (1e):Verbindung 1e wird analog zu 3a synthetisiert.

### Beispiel 5

a) Synthese von N-Carbonyl-4-carboxypiperidyl-(1→2)-[(L-threityl)-(1→1)]-(1R,2R)-trans-1,2-cyclohexandiol (1f):
   Verbindung 1f wird analog zu 3a hergestellt wobei (1R,2R)-trans-1,2-cyclohexandiol nicht mit Thioethyl-O-2,3,4-tri-O-benzyl-α-L-fucopyranosid sondern mit 2,3,4-tri-O-benzyl-1-O-toluolsulfonyl-L-threitol (in Toluol/50 % Natronlauge und Tetrabutylammoniumbromid als Phasentransferkatalysator) umgesetzt wird (s. a. Beispiel 7).

### Beispiel 6

c) Synthese von [N-Carbonyl-(1,1-dicarboxy-ethyl)-(2→2)-(R oder S)-pyrrolidyl]-(1→2)-[(α-L-fucopyranosyl)-(1→1)]-(1R,2R)-trans-1,2-cyclohexandiol (1g):
   Verbindung 1g wird analog zu 4b hergestellt wobei 1a nicht mit 3-(Hydroxymethyl)-piperidin sondern mit Prolinol (D oder L) umgesetzt wird.

### Beispiel 7

a) Synthese von N-Carbonyl-4-carboxypiperidyl-(1→2)-[(methyl-α-Dmannopyranosyl)- (6→1)]-(1R,2R)-trans-1,2-cyclohexandiol (1h):
   Zu einer Mischung aus (1R,2R)-trans-1,2-Cyclohexandiol (336 mg, 2,894 mmol), Toluol (8 ml), Tetrabutylammoniumbromid (311 mg, 0,97 mmol) und 50 %iger Natronlauge (4,5 ml) gibt man eine Lösung aus Methyl-2,3.4-tri-O-benzyl-6-O-toluolsulfonyl-α-D-mannopyranosid (1,19 g, 1,93 mmol) in Toluol (10 ml). Es wird 12 h bei 60°C gerührt, mit Ether verdünnt und mit Wasser neutral gewaschen. Flash-chromatographie (Toluol/Aceton 6:1 - 5:1) liefert [(Methyl-2,3,4-tri-O-benzyl-α-D-mannopyranosyl)-(6→1)]-(1R,2R)-trans-1,2-cyclohexandiol, welches wie Verbindung 1a aus Beispiel 1 weiterverarbeitet wird.

### Beispiel 8

a) Synthese von N-Carbonyl-4-carboxypiperidyl-(1→2)-[(methyl-β-D-galactopyranosyl)-6→1)]-(1R,2R)-trans-1,2-cyclohexandiol (1i):
   Verbindung 1i wird analog zu 1h (Beispiel 7) hergestellt, wobei (1R,2R)-trans-1,2-cyclohexandiol mit Methyl-2,3,4-tri-O-benzyl-6-O-toluolsulfonyl-β-D-galactopyranosid in Toluol/50 % Natrolauge mit Tetrabutylammoniumbromid als Phasentransferkatalysator umgesetzt und die geschützte Vorstufe wie 1a aus Beispiel 1 weiterverarbeitet wird.

### Beispiel 9

a) Synthese von N-Carbonyl-4-carboxypiperidyl-(1→2)-[galactopyranosyl-(6→1)]-(1R,2R)-trans-1,2-cyclohexandiol (1k):
   Verbindung 1k wird analog zu 1h (Beispiel 8) hergestellt wobei (1R,2R)-trans-1,2- cyclohexandiol mit Benzyl-2,3,4-tri-O-benzyl-6-O-trifluormethansulfonyl-β-D-galactopyranosid (in Dimethylformamid/1,2 Äquivalente Natriumhydrid) umgesetzt wird.

## Patentansprüche

1. Verbindung der Formel I 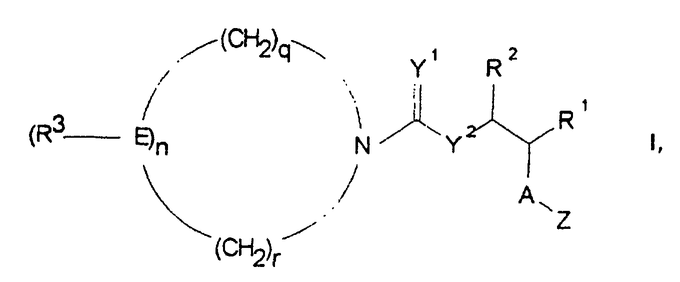 worin bedeuten
Z ein Pyranosid, ein über die C6-Position verknüpfter Pyranosylrest, ein über die C6-Position verknüpftes Alkyl-Pyranosid, ein Furanosid, ein über die C5-Position verknüpfter Furanosylrest, ein über die C5-Position verknüpftes Alkyl-Furanosid oder ein Polyalkohol, welcher über eine beliebige Position mit A verknüpft ist,
A Sauerstoff, -CH₂- oder Schwefel,
R¹ und R² unabhängig voneinander Wasserstoff, -(CH₂)ₘX¹ oder CH₂O(CH₂)ₘX², wobei m eine ganze Zahl von 1 bis 20 bedeutet, oder gemeinsam einen fünf- oder sechsgliedrigen Carbo- oder Heterocyclus mit mindestens einem der Substituenten R⁴, R⁵ oder R⁶,
E Stickstoff, Kohlenstoff oder -CH-,
R³ -(CH₂)ₚCOOH, (-COOH)₂, -(CH₂)ₚCH(COOH)₂, -(CH₂)ₚCNH₂(COOH)₂, -(CH₂)ₚC(CH₂-C₆H₅)(COOH)₂, -CONHC(COOH)₂, wobei p eine ganze Zahl von 0 bis 10 bedeutet, oder 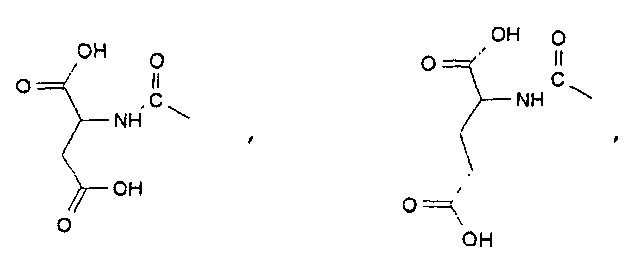 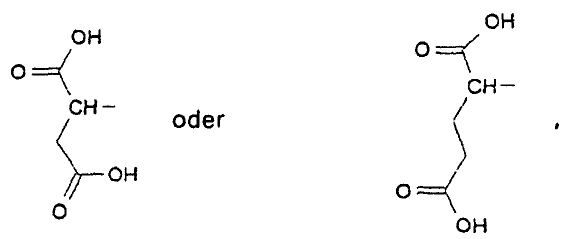
q und r unabhängig voneinander eine ganze Zahl von 0 bis 3,
n eine ganze Zahl von 1 bis 3 mit der Maßgabe, daß die Summe von q, r und n 4 oder 5 beträgt,
R⁴, R⁵ und R⁶ unabhängig voneinander H, OH, -O(CH₂)_{w}X³ oder CH₂O(CH₂)_{w}X⁴, wobei
w eine ganze Zahl von 1 bis 18 bedeutet,
Y¹ und Y² unabhängig voneinander Sauerstoff, -NH- oder Schwefel und
X¹, X², X³ und X⁴ unabhängig voneinander Wasserstoff, -NH₂, -COOH, -OH, -CH₂OH, CH₂NH₂, -C₁-C₂₀-Alkyl oder -C₆-C₁₀-Aryl.

2. Verbindung nach Anspruch 1, **dadurch gekennzeichnet, daß** R¹ und R² gemeinsam einen Cyclohexanring bilden.

3. Verbindung nach Anspruch 1, **dadurch gekennzeichnet, daß** R¹ und R² gemeinsam einen Cyclopentanring bilden.

4. Verbindung nach einem der Ansprüche 1 bis 3, **dadurch gekennzeichnet, daß** A Sauerstoff bedeutet.

5. Verbindung nach einem der Ansprüche 1 bis 4, **dadurch gekennzeichnet, daß** Y¹ und Y² Sauerstoff bedeuten.

6. Verbindung nach einem der Ansprüche 1 bis 5, **dadurch gekennzeichnet, daß**
n 1 und
q und r 2 bedeuten.

7. Verbindung nach einem der Ansprüche 1 bis 5, **dadurch gekennzeichnet, daß**
n und r 1 und
q 3 bedeuten.

8. Verbindung nach einem der Ansprüche 1 bis 5, **dadurch gekennzeichnet, daß**
n 1,
q 0 und
r 3 bedeuten.

9. Verbindung nach einem der Ansprüche 1 bis 8, **dadurch gekennzeichnet, daß** E -CH-, R³ -(CH₂)ₚCOOH und p 0 bedeuten.

10. Verbindung nach einem der Ansprüche 1 bis 8, **dadurch gekennzeichnet, daß** E -CH-, R³ -(CH₂)ₚCH(COOH)₂ und p 1 bedeuten.

11. Verbindung nach einem der Ansprüche 1 bis 8, **dadurch gekennzeichnet, daß** E Kohlenstoff und R³ (-COOH)₂ bedeuten.

12. Verbindung nach einem der Ansprüche 1 bis 11, **dadurch gekennzeichnet, daß** Z ein Pyranosid ist.

13. Verbindung nach Anspruch 12, **dadurch gekennzeichnet, daß** das Pyranosid ein L-Fucosid ist.

14. Verbindung nach Anspruch 12, **dadurch gekennzeichnet, daß** das Pyranosid ein D-Mannosid ist.

15. Verbindung nach Anspruch 12, **dadurch gekennzeichnet, daß** das Pyranosid ein L-Rhamnosid ist.

16. Verbindung nach Anspruch 12, **dadurch gekennzeichnet, daß** das Pyranosid ein L-Galactosid ist.

17. Verbindung nach Anspruch 12, **dadurch gekennzeichnet, daß** das Pyranosid ein L-Mannosid ist.

18. Verbindung nach einem der Ansprüche 1 bis 11, **dadurch gekennzeichnet, daß** Z ein Furanosid ist.

19. Verbindung nach Anspruch 18, **dadurch gekennzeichnet, daß** das Furanosid ein Ribosid ist.

20. Verbindung nach einem der Ansprüche 1 bis 11, **dadurch gekennzeichnet, daß** Z ein über die C6-Position verknüpfter D-Mannosylrest ist.

21. Verbindung nach einem der Ansprüche 1 bis 11, **dadurch gekennzeichnet, daß** Z ein über die C6-Position verknüpftes Methyl-D-Mannosid ist.

22. Verbindung nach einem der Ansprüche 1 bis 11, **dadurch gekennzeichnet, daß** Z ein L-Threit-1-yl-Rest ist.

23. Verfahren zur Herstellung einer Verbindung der Formel I gemäß einem der Ansprüche 1 bis 22, **dadurch gekennzeichnet, daß** man zunächst unter O- oder S-Glycosylierung, Alkylierung oder C-C-Verknüpfung einer funktionellen Gruppe eines Akzeptors der Formel II, 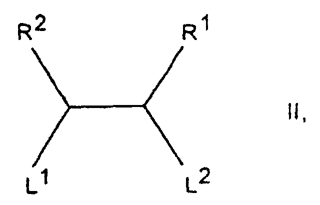 aufweisend mindestens zwei benachbarte funktionelle Gruppen L¹ und L² sowie aufweisend die Substituenten R¹ und R², mittels eines Äquivalents eines mit einer aktivierenden Gruppe L³ und gegebenenfalls mit Schutzgruppen versehenen Donors der Formel III,
Z ― L³ III,
Zwischenverbindung IV 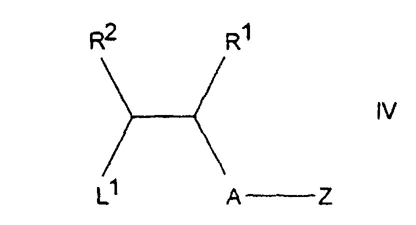 herstellt, wonach man unter Umsetzung mit Reagenz der Formel V, 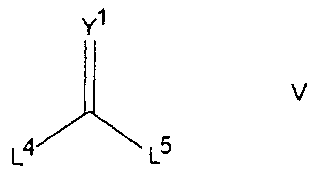 worin L⁴ und L⁵ die Bedeutung von Abgangsgruppen haben, zu der aktivierten Verbindung VI, 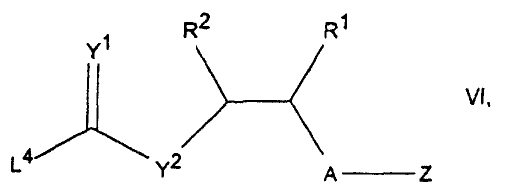 gelangt, welche durch Umsetzung mit einem einfach oder mehrfach carboxylierten cyclischen Amin oder einer geeigneten Vorstufe davon und gegebenenfalls nach Cyclisierung oder Carboxylierung sowie nach Abspaltung von Schutzgruppen zu der Verbindung der Formel I überführt wird, wobei die Variablen R¹, R², Y¹, Y², A und Z die in Anspruch 1 genannte Bedeutung haben.

24. Arzneimittel enthaltend wenigstens eine Verbindung gemäß einem der Ansprüche 1 bis 22.

25. Verwendung einer Verbindung gemäß einem der Ansprüche 1 bis 22 für die Herstellung eines Arzneimittels zur Therapie oder Prophylaxe einer Krankheit, welche mit einer übermäßigen, durch Selektinrezeptoren vermittelten Zelladhäsion in dem von der Krankheit betroffenen Gewebe einhergeht.

26. Verwendung nach Anspruch 25, **dadurch gekennzeichnet, daß** die Krankheit eine Herz-Kreislauf-Erkrankung ist.

27. Verwendung einer Verbindung gemäß einem der Ansprüche 1 bis 22 für die Herstellung eines Mittels zur Diagnose einer Krankheit, welche mit einer übermäßigen, durch Selektinrezeptoren vermittelten Zelladhäsion in dem von der Krankheit betroffenen Gewebe einhergeht.

## Revendications

1. Composé de formule I 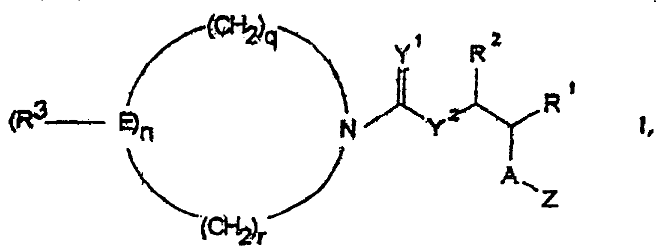 dans laquelle
Z est un pyranoside, un résidu pyranosyle lié via la position C₆, un alkyl-pyranoside lié via la position C₆, un furanoside, un résidu furanosyle lié via la position C₅, un alkylfuranoside lié via la position C₅ ou un polyol qui est lié à A via une position quelconque.
A est l'oxygène, -CH₂- ou le soufre,
R¹ et R² représentent, indépendamment l'un de l'autre, l'hydrogène, -(CH₂)ₘX¹ ou CH₂O(CH₂)ₘX², où m est un nombre entier comptis entre 1 et 20, ou représentent conjointement un carbo- ou un hétérocycle à cinq ou six chaînons et comportant au moins un des substituants R⁴,R⁵ ou R⁶,
E est l'azote, le carbone ou -CH-,
R³ est -(CH₂)ₚCOOH, (-COOH)₂, -(CH₂)ₚCH(COOH)₂, -(CH₂)ₚCNH₂(COOH)₂, -(CH₂)ₚC(CH₂-C₆H₅)(COOH)₂, -CONHC(COOH)₂, où p est un nombre entier compris entre 0 et 10, ou 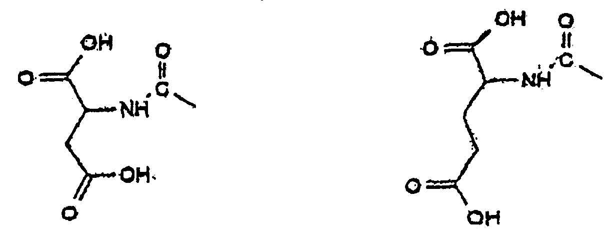 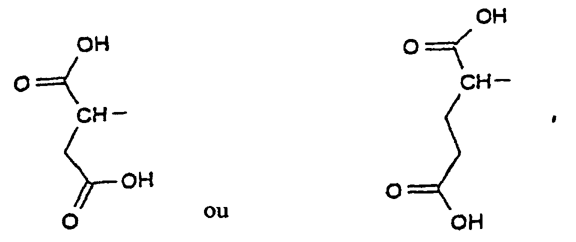
q et r sont, indépendamment l'un de l'autre, un nombre entier compris entre 0 et 3,
n est un nombre entier compris entre 1 et 3, à la condition que la somme de q, r et n soit égale à 4 ou 5,
R⁴,R⁵ et R⁶ représentent, indépendamment l'un de l'autre, H, OH, -O(CH₂)_{w}X³ ou CH₂O(CH₂)_{w}X⁴, où w est un nombre entier compris entre 1 et 18,
Y¹ et Y² représentent, indépendamment l'un de l'autre l'oxygène, -NH- ou le soufre, et
X¹,X² X³ et X⁴ représentent, indépendamment l'un de l'autre, l'hydrogène, -NH₂, -COOH, -OH, -CH₂OH, CH₂NH₂, -alkyle en C₁ à C₂₀ ou -aryle en C₆ à C₁₀.

2. Composé selon la revendication 1, **caractérisé en ce que** R¹ et R² forment conjointement un cycle cyclohexane.

3. Composé selon la revendication 1, **caractérisé en ce que** R¹ et R² forment conjointement un cycle cyclopentane.

4. Composé selon l'une des revendications 1 à 3, **caractérisé en ce que** A représente l'oxygène.

5. Composé selon l'une des revendications 1 à 4, **caractérisé en ce que** Y¹ et Y² représentent l'oxygène.

6. Composé selon l'une des revendications 1 à 5, **caractérisé en ce que**
n vaut 1 et
q et r valent 2.

7. Composé selon l'une des revendications 1 à 5, **caractérisé en ce que**
n et r valent 1 et
q vaut 3.

8. Composé selon l'une des revendications 1 à 5, **caractérisé en ce que**
n vaut 1,
q vaut 0 et
r vaut 3.

9. Composé selon l'une des revendications 1 à 8, **caractérisé en ce que** E est -CH-, R³ est -(CH₂)ₚCOOH et p vaut 0.

10. Composé selon l'une des revendications 1 à 8, **caractérisé en ce que** E est -CH-, R³ est -(CH₂)ₚCH(COOH)₂ et p vaut 1.

11. Composé selon l'une des revendications 1 à 8, **caractérisé en ce que** E est le carbone et R³ est (-COOH)₂.

12. Composé selon l'une des revendications 1 à 11, **caractérisé en ce que** Z est un pyranoside.

13. Composé selon la revendication 12, **caractérisé en ce que** le pyranoside est un L-fucoside.

14. Composé selon la revendication 12, **caractérisé en ce que** le pyranoside est un D-mannoside.

15. Composé selon la revendication 12, **caractérisé en ce que** le pyranoside est un L-rhamnoside.

16. Composé selon la revendication 12, **caractérisé en ce que** le pyranoside est un L-galactoside.

17. Composé selon la revendication 12, **caractérisé en ce que** le pyranoside est un L-mannoside.

18. Composé selon l'une des revendications 1 à 11, **caractérisé en ce que** Z est un furanoside.

19. Composé selon la revendication 18, **caractérisé en ce que** le furanoside est un riboside.

20. Composé selon l'une des revendications 1 à 11, **caractérisé en ce que** Z est un résidu D-mannosyle lié via la position C₆.

21. Composé selon l'une des revendications 1 à 11, **caractérisé en ce que** Z est un méthyl-D-mannoside lié via la position C₆.

22. Composé selon l'une des revendications 1 à 11, **caractérisé en ce que** Z est un résidu L-thréit-1-yle.

23. Procédé de préparation d'un composé de formule 1 selon l'une des revendications 1 à 22, **caractérisé en ce que** l'on prépare d'abord, par O- ou S-glycosylation, alkylation ou liaison C-C d'un groupe fonctionnel d'un accepteur de formule II, 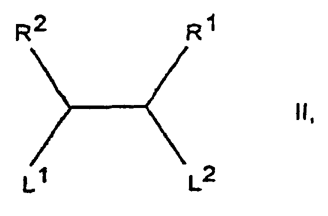 présentant au moins deux groupes fonctionnels adjacents L¹ et L² et présentant les substituants R¹ et R², au moyen d'un équivalent d'un donneur de formule III, pourvu d'un groupe activant L³ et éventuellement de groupes protecteurs,
Z ― L³ III,
le composé intermédiaire, IV 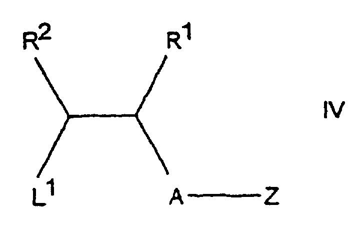 suite à quoi on obtient, par transformation avec un réactif de formule V, 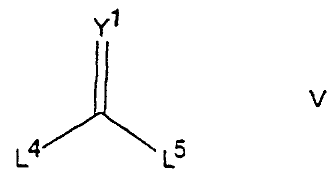 dans laquelle L⁴ et L⁵ ont la signification de groupes partants, le composé activé VI, 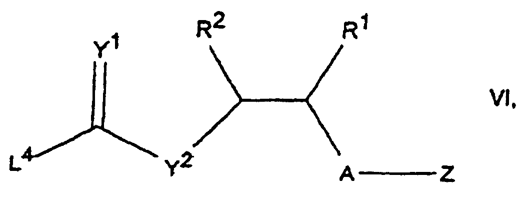 qui, par conversion avec une amine cyclique mono- ou polycarboxylée ou un précurseur approprié de celle-ci, et éventuellement après cyclisation ou carboxylation, ainsi qu'après séparation des groupes protecteurs, est transformé en composé de formule I, les variables R¹, R², Y¹, Y², A et Z ayant la signification nommée dans la revendication 1.

24. Médicament contenant au moins un composé selon l'une des revendications 1 à 22.

25. Utilisation d'un composé selon l'une des revendications 1 à 22 pour fabriquer un médicament destiné à la thérapie ou à la prophylaxie d'une maladie qui est liée à une adhésion cellulaire excessive, induite par les récepteurs de sélectine, dans le tissu touché par la maladie.

26. Utilisation selon la revendication 25, **caractérisée en ce que** la maladie est une maladie cardio-vasculaire.

27. Utilisation d'un composé selon l'une des revendications 1 à 22 pour fabriquer un moyen de diagnostic d'une maladie qui est liée à une adhésion cellulaire excessive, induite par les récepteurs de sélectine, dans le tissu touché par la maladie.

## Claims

1. A compound of formula I 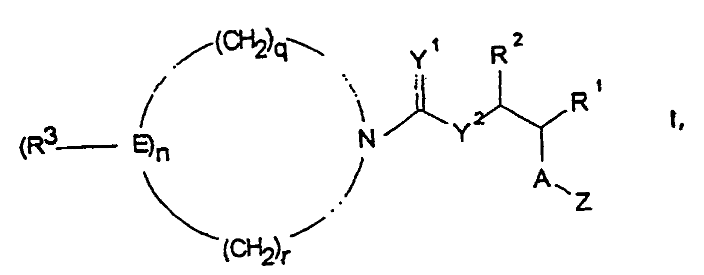 wherein
Z is a pyranoside, a pyranosyl residue linked via the C6 position, an alkyl pyranoside linked via the C6 position, a furanoside, a furanosyl residue linked via the C5 position, an alkyl furanoside linked via the C5 position, or a polyalcohol which is linked via any position to A,
A is oxygen, -CH₂- or sulfur,
R¹ and R² independently of one another are hydrogen, -(CH₂)ₘX¹ or CH₂O(CH₂)ₘX², where m is an integer of 1 to 20, or together a five- or six-membered carbo- or heterocycle having at least one of the substituents R⁴, R⁵, or R⁶,
E is nitrogen, carbon, or -CH-,
R³ is (CH₂)ₚCOOH, (-COOH)₂, -(CH₂)ₚCH(COOH)₂, -(CH₂)ₚCNH₂(COOH)₂, -(CH₂)ₚC(CH₂-C₆H₅)(COOH)₂, -CONHC(COOH)₂, where p is an integer of 0 to 10, or 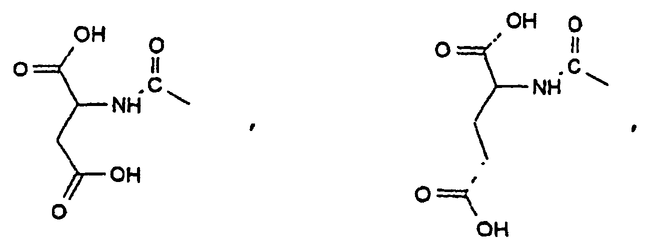 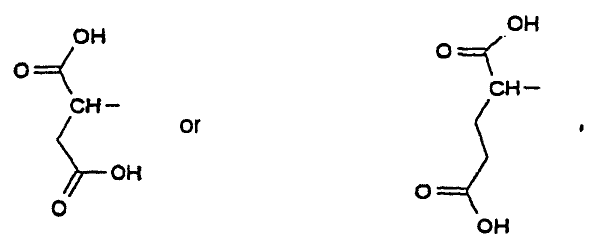
q and r independently of one another are an integer of 0 to 3,
n is an integer of 1 to 3 with the proviso that the sum of q, r and n is 4 or 5,
R⁴, R⁵, and R⁶ independently of one another are H, OH, -O(CH₂)_{w}X³, or CH₂O(CH₂)_{w}X⁴, where
w is an integer of 1 to 18,
Y¹ and Y² independently of one another are oxygen, -NH- or sulfur, and
X¹, X², X³ and X⁴ independently of one another are hydrogen, -NH₂, -COOH, -OH, -CH₂OH, CH₂NH₂, -C₁-C₂₀ alkyl, or -C₆-C₁₀ aryl.

2. Compound according to claim 1, **characterized in that** R¹ and R² together form a cyclohexane ring.

3. Compound according to claim 1, **characterized in that** R¹ and R² together form a cyclopentane ring.

4. Compound according to any one of claims 1 to 3, **characterized in that** A is oxygen.

5. Compound according to any one of claims 1 to 4, **characterized in that** Y¹ and Y² are oxygen.

6. Compound according to any one of claims 1 to 5, **characterized in that**
n is 1 and
q and r are 2.

7. Compound according to any one of claims 1 to 5, **characterized in that**
n and r are 1 and
q is 3.

8. Compound according to any one of claims 1 to 5, **characterized in that**
n is 1,
q is 0, and
r is 3.

9. Compound according to any one of claims 1 to 8, **characterized in that** E is -CH-, R³ is -(CH₂)ₚCOOH, and p is 0.

10. Compound according to any one of claims 1 to 8, **characterized in that** E is -CH-, R³ is -(CH₂)ₚCH(COOH)₂, and p is 1.

11. Compound according to any one of claims 1 to 8, **characterized in that** E is carbon and R³ is (-COOH)₂.

12. Compound according to any one of claims 1 to 11, **characterized in that** Z is a pyranoside.

13. Compound according to claim 12, **characterized in that** the pyranoside is an L-fucoside.

14. Compound according to claim 12, **characterized in that** the pyranoside is a D-mannoside.

15. Compound according to claim 12, **characterized in that** the pyranoside is an L-rhamnoside.

16. Compound according to claim 12, **characterized in that** the pyranoside is an L-galactoside.

17. Compound according to claim 12, **characterized in that** the pyranoside is an L-mannoside.

18. Compound according to any one of claims 1 to 11, **characterized in that** Z is a furanoside.

19. Compound according to claim 18, **characterized in that** the furanoside is a riboside.

20. Compound according to any one of claims 1 to 11, **characterized in that** Z is a D-mannosyl residue linked via the C6 position.

21. Compound according to any one of claims 1 to 11, **characterized in that** Z is a methyl-D-mannoside linked via the C6 position.

22. Compound according to any one of claims 1 to 11, **characterized in that** Z is an L-threitol-1-yl residue.

23. A method for preparing a compound of formula I according to any one of claims 1 to 22, **characterized in that** first under O- or S-glycosylation, alkylation or CC linkage of a functional group of an acceptor of formula II, 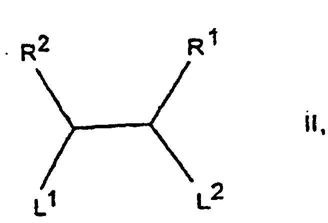 comprising at least two adjacent functional groups L¹ and L², and comprising the substituents R¹ and R², there is prepared by means of an equivalent of a donor provided with an activating group L³ and optionally with protective groups, according to formula III,
Z ― L³ III,
an intermediate compound IV 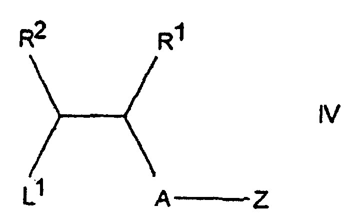 after which under reaction with a reagent of formula V 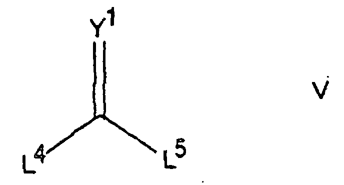 where L⁴ and L⁵ have the meaning of leaving groups, there is obtained the activated compound VI, 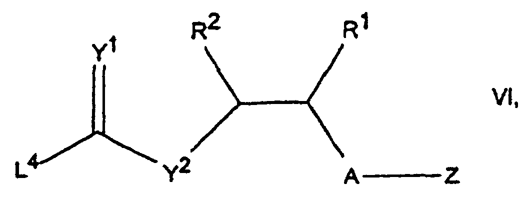 which is converted by reaction with a singly or multiply carboxylated cyclic amine, or a suitable precursor thereof, and optionally after cyclization or carboxylation and after cleavage of protective groups, to the compound of formula I, the variables R¹, R², Y¹, Y², A and Z having the meaning indicated in claim 1.

24. A medicament containing at least one compound according to any one of claims 1 to 22.

25. Use of a compound according to any one of claims 1 to 22 for preparing a medicament for the therapy or prophylaxis of a disease which is accompanied by an excessive cell adhesion mediated by selectin receptors in the tissue affected by the disease.

26. Use according to claim 25, **characterized in that** the disease is a cardiovascular disease.

27. Use of a compound according to any one of claims 1 to 22 for producing an agent for diagnosing a disease which is accompanied by an excessive cell adhesion mediated by selectin receptors in the tissue affected by the disease.
